# EUROPÄISCHE PATENTSCHRIFT

(11) **EP 2 943 174 B1**
(45) Veröffentlichungstag und Bekanntmachung des Hinweises auf die Patenterteilung: **02.05.2018**
(21) Anmeldenummer: 14709526.9
(22) Anmeldetag: 10.01.2014
(51) Int. Cl.: A61G 11/00, A61G 7/005

(54) **HEIZUNG FÜR EINEN INKUBATOR FÜR KLEINKINDER UND INKUBATOR FÜR KLEINKINDER**
HEATER FOR AN INCUBATOR FOR INFANTS AND INCUBATOR FOR INFANTS
SYSTÈME DE CHAUFFAGE DESTINÉ À UNE COUVEUSE POUR NOURRISSON ET COUVEUSE POUR NOURRISSON

(30) Priorität: 14.01.2013 DE 102013000476
(43) Veröffentlichungstag der Anmeldung: 18.11.2015
(73) Patentinhaber: Drägerwerk AG & Co. KGaA, 23558 Lübeck (DE)
(72) Erfinder: KOCH, Jochim, 23909 Ratzeburg (DE); LISCHINSKI, Robert, 23911 Einhaus (DE); SCHMID, Uwe, 23564 Lübeck (DE)
(74) Vertreter: Kettenbeil, Roxane Henriette
(86) Internationale Anmeldenummer: PCT/DE2014/000010
(87) Internationale Veröffentlichungsnummer: WO 2014/108126

(56) Entgegenhaltungen:
- WO-A1-98/48755
- DE-A1- 1 566 436
- US-A- 5 616 115

## Beschreibung

Die Erfindung betrifft eine Heizung für einen Inkubator für Kleinkinder, gemäß dem Oberbegriff von Anspruch 1 sowie einen Inkubator für Kleinkinder mit einer solchen Heizung.

Inkubatoren für Kleinkinder, insbesondere für Neu- und Frühgeborene (Neonaten) sind allgemein bekannt. Sie spielen vor allem im intensivmedizinischen Bereich eine wichtige Rolle, etwa bei der Unterstützung des Entwicklungs- und Wachstumsprozesses von Neonaten. Mit Hilfe solcher Inkubatoren wird üblicherweise sichergestellt, dass das zu behandelnde Kind kontrollierten Umgebungsbedingungen ausgesetzt wird. Erfindungsgemäße Inkubatoren werden auch als Wärmtherapiegeräte bezeichnet. Dabei sind sowohl geschlossene als auch offene Geräte bekannt. Geschlossene Geräte zeichnen sich üblicherweise durch das Vorhandensein einer verschwenkbaren oder aufklappbaren Abdeckhaube aus. Bei offenen Geräten fehlt eine solche Abdeckhaube zumeist. Bei sogenannten Hybridgeräten kann eine vorhandene Abdeckhaube abgenommen oder geöffnet werden, so dass ein und dasselbe Gerät sowohl im offenen als auch im geschlossenen Pflegemodus betrieben werden kann. Besonders wichtig ist dabei in jedem Fall die Regelung der Temperatur, die im Inkubator herrscht. Inkubatoren für Kleinkinder sind daher üblicherweise beheizt.

Die Beheizung solcher Inkubatoren - insbesondere im geschlossenen Pflegemodus - kann beispielsweise mit Hilfe einer Konvektionsheizung erfolgen. Dazu weist der Inkubator bzw. die Heizung des Inkubators zumeist ein Lüfterrad auf, das eine Umwälzung der Innenraumluft bewirkt. Die umgewälzte Innenraumluft strömt dann über ein Wärmetauscherelement, bevor sie in den Bereich gelangt, in welchem sich das zu behandelnde Kind befindet. Typische Wärmetauscherelemente (Wärmeübertragungselemente) einer solchen Heizung können beispielsweise Heizwendelrohre oder rippenförmige Heizkörper sein. Insbesondere Heizwendelrohre haben sich jedoch in Bezug auf die Wärmeübertragung, Anordnung im Strömungskanal und bei der Reinigung als nicht unbedingt optimal herausgestellt. Man hat daher versucht, andere Heizungen für Inkubatoren zu entwickeln.

US 6,428,465 sieht beispielsweise einen Inkubator mit einer Heizung vor, bei welcher ein oberes und ein unteres Gehäuseteil einen Strömungskanal (Strömungskammer) bilden. Dieser Strömungskanal umfasst eine obere Luftkammer und eine untere Luftkammer. Das Lüfterrad ist zentrisch in der Heizung des Inkubators angeordnet. Der Einlass der Luft in die Strömungskammer erfolgt durch Einlassluftbereiche, die sich an den Stirnseiten des Inkubators befinden. Der Auslass der Luft erfolgt durch Auslassluftkanäle, die sich an den Längsseiten des Inkubators befinden. Insgesamt entstehen auf diese Weise ein Einlassluftbereich in der unteren Luftkammer, in welchem die Luft zum Lüfterrad hin strömt, und ein quer dazu angeordneter Auslassluftbereich in der oberen Luftkammer, in welchem die Luft vom Lüfterrad wieder weg strömt. Das Lüfterrad ist derart am Übergang zwischen der unteren und der oberen Luftkammer angeordnet, dass es bei Betrieb des Inkubators in der unteren. Luftkammer eine ansaugende und in der quer dazu angeordneten oberen Luftkammer eine wegblasende Wirkung auf den Luftstrom hat.

Zur Beheizung des Luftstromes ist bei der US 6,428,465 ein Wärmetauscherelement vorgesehen, welches mit einer Vielzahl von Lamellen versehen ist. Diese Lamellen dienen der Erhöhung der Wärmetauschfläche und der Strömungsführung und erstrecken sich von der Oberseite des Wärmetauscherelementes mehr oder weniger senkrecht nach oben. Das Wärmetauscherelement ist in der unteren Luftkammer angeordnet, d.h. im Einlassluftbereich, in welchem die einströmende Luft zum Lüfterrad hinströmt. Der Auslassluftbereich ist nicht beheizt.

US 5,935,055 sieht ebenfalls eine verbesserte Heizung für einen Inkubator mit einem zentral angeordneten Lüfterrad vor. Die aus der Inkubatorkammer in den Strömungsbereich der Heizung einströmende Luft strömt von den Stirnseiten aus zu diesem zentralen Lüfterrad und wird dort mit Hilfe eines ebenfalls zentrischen, ringförmigen Wärmetauscherelementes erhitzt. Die Luft wird anschließend an den beiden Längsseiten des Inkubators zurück in die Inkubatorkammer ausgeströmt.

Problematisch ist in beiden Fällen, dass die Heizung durch die relativ komplizierte Geometrie der jeweiligen Wärmetauscherelemente - beispielsweise im Bereich der Lamellen - sehr aufwendig und umständlich zu reinigen sein kann. Insbesondere können dabei Stellen in der Heizung vorhanden sein, die einer vollständigen und sachgemäßen Reinigung nur schwer zugänglich sind.

Außerdem kann die Notwendigkeit bestehen, die jeweilige Heizung, insbesondere das jeweilige Wärmetauscherelement, mit einer relativ hohen Oberflächentemperatur zu betreiben, damit eine ausreichende und gleichmäßige Erwärmung der Luft sichergestellt wird. So kann die Oberflächentemperatur beispielsweise den Wert von 100°C leicht und deutlich übersteigen. Das gilt insbesondere auch für Heizwendeln, die eine relativ geringe Oberfläche haben können.

Ein weiteres Problem können die in den Strömungsgang eingesetzten Elemente - beispielsweise die Lamellen oder die zentrische, ringförmige Heizung - aus akustischer Sicht darstellen. Durch diese Elemente kann es zu einer nicht unerheblichen Geräuschentwicklung kommen.

Ausgehend davon ist es Aufgabe der Erfindung, eine verbesserte Heizung für einen Inkubator für Kleinkinder bereitzustellen, der diese und andere Nachteile des Standes der Technik überwindet.

Darüber hinaus soll ein entsprechender Inkubator für Kleinkinder mit einer verbesserten Heizung bereitgestellt werden. Insbesondere soll eine Heizung für einen Inkubator für Kleinkinder bereitgestellt werden, die einfach zu reinigen und möglichst geräuscharm im Betrieb ist. Weiterhin soll die Heizung mit einer möglichst geringen Oberflächentemperatur betrieben werden können. Gleichzeitig soll eine gleichmäßige Lufterwärmung möglich sein.

Zur Lösung dieser Aufgabe sieht die Erfindung einen Inkubator für Kleinkinder mit den Merkmalen des unabhängigen Anspruchs 1 vor. Weitere Ausgestaltungen sind jeweils Gegenstand der abhängigen Ansprüche 2 bis 15.

Bei einer Heizung für einen Inkubator für Kleinkinder, wobei die Heizung eine Gehäusestruktur mit einer oberen Gehäusefläche und einer unteren Gehäusefläche, wenigstens einen Lufteinlass für einen anströmenden Luftstrom, wenigstens einen Luftauslass für einen ausströmenden Luftstrom, wenigstens ein Lüfterrad, wenigstens ein Heizelement und wenigstens ein Wärmeübertragungselement aufweist und wobei die obere Gehäusefläche und die untere Gehäusefläche einen Strömungsraum begrenzen, sieht die Erfindung vor, dass das Wärmeübertragungselement eine bei Betrieb der Heizung horizontal angeordnete Platte mit einer ebenen Oberfläche ist.

Eine Heizung im Sinne der vorliegenden Erfindung kann alternativ auch als Heizvorrichtung oder als Vorrichtung zur Beheizung der Innenraumluft eines Inkubators für Kleinkinder bezeichnet werden.

Bevorzugt ist das Wärmeübertragungselement dabei eine Platte, deren Größe bevorzugt ganz oder teilweise der Grundfläche der Heizung entspricht. Die Grundfläche der Heizung ist dabei die Projektionsfläche der Heizung bei einer senkrechten Aufsicht von oben auf die im Betrieb befindliche - d.h. im Inkubator angeordnete - Heizung. Weiterhin entspricht die Form der Platte ganz oder teilweise der Form der obengenannten Projektionsfläche der Heizung. Die Form der Platte ist dabei unabhängig von ihrer Größe, d.h. die Platte kann beispielsweise die selbe Form wie die Projektionsfläche der Heizung aber eine andere Größe aufweisen und umgekehrt. Eine entsprechende Platte hat eine prinzipiell flache, quaderähnliche Gestalt mit jeweils einem Paar von einander gegenüberliegenden Hauptflächen, einem Paar langen Seitenflächen und einem Paar kurzen Seitenflächen. Dabei sind die Seitenflächen im Vergleich zu den Hauptflächen sehr schmal ausgebildet. Selbstverständlich können die langen und die kurzen Seitenflächen auch die gleiche Länge aufweisen, so dass die Hauptflächen eine mehr oder weniger quadratische Gestalt haben. Bevorzugt haben die Seitenflächen lediglich die Gestalt von Kanten, die die Hauptflächen begrenzen. Die durch die Seitenflächen gebildeten Kanten können eckig sein. Es sind aber auch runde oder geschwungene Übergänge zwischen den Flächen vorstellbar. Auch können alle Flächen Krümmungen oder Wölbungen aufweisen, die gleichförmig oder unterschiedlich groß sein können. Es ist beispielsweise denkbar, dass die Platte derart gewölbt ist, dass sie eine wannenförmige Form aufweist. Auch eine halbellipsoide Form ist vorstellbar. Mit anderen Worten, die Platte kann eine zumindest teilweise gewölbte Platte mit einer ebenen Oberfläche, bevorzugt mit einer lamellenfreien Oberfläche wie nachfolgend beschrieben, sein.

Die Hauptflächen erstrecken sich im Betrieb der Heizung bevorzugt in horizontaler Richtung, d.h. parallel zur Grundfläche der Heizung. In jedem Fall ist die Hauptfläche einer solchen Platte, mithin also des Wärmeübertragungselementes, derart ausgebildet und in der Heizung angeordnet, dass sie sich im Betrieb der Heizung parallel zur Liegefläche eines erfindungsgemäßen, unten beschriebenen Inkubators erstrecken kann.

Mit anderen Worten, das Wärmeübertragungselement ist plattenförmig - bevorzugt mit einer ungefähr rechteckigen Hauptfläche mit optional abgerundeten Ecken - und weist eine im Wesentlichen unstrukturierte, glatte Oberfläche auf. Dabei wird unter einer "ebenen Oberfläche" oder unter einer "im Wesentlichen unstrukturierten, glatten" Oberfläche verstanden, dass das Wärmeübertragungselement keine Lamellen oder ähnlichen Vorsprünge oder Erhebungen aufweist. Mit anderen Worten, eine ebene Oberfläche kann eine lamellenfreie Oberfläche sein. Insbesondere weist das Wärmeübertragungselement keine Elemente auf, die sich von der Grundfläche des Wärmeübertragungselementes aus nach oben erstrecken und die im Betrieb der Heizung geeignet sind, Verwirbelungen eines über die Platte strömenden Luftstromes zu erzeugen, welche zu Strömungsgeräuschen führen, die für das menschliche Ohr, insbesondere für Neonaten, hörbar sind. Mithin kann das Erzeugen von Strömungsgeräuschen vermieden werden, die von dem in dem Inkubator liegenden Kind wahrgenommen werden können.

Man erkennt in diesem Zusammenhang, dass das Wärmeübertragungselement bevorzugt lamellenfrei ist. Besonders bevorzugt ist es frei von Lamellen, die bei Betrieb der Heizung senkrecht orientiert sind und eine Führung des Luftstromes bewirken. Eine solche glatte Platte mit einer ebenen Oberfläche ist leicht abwischbar und daher gut zu reinigen. Zudem werden so gut wie keine störenden Verwirbelungen oder Geräusche erzeugt, wenn ein Luftstrom über die Platte, d.h. also das Wärmeübertragungselement, hinwegströmt.

Das Wärmeübertragungselement besteht vorteilhafterweise aus einem gut wärmeleitfähigen Material, beispielsweise Metall, temperaturbeständigem und gut wärmeleitendem Kunststoff oder dergleichen. Dabei kann das Wärmeübertragungselement eine massive Platte sein, die aus dem wärmeleitenden Material besteht. Es ist jedoch auch vorstellbar, dass das Wärmeübertragungselement einen hohlen Kern aufweist, der als Fluidkammer dienen kann. Durch eine solche Fluidkammer kann ein erwärmtes Fluid, beispielsweise Wasser oder Luft, strömen. In jedem Fall ist die Oberfläche des Wärmeübertragungselementes aufheizbar und das Wärmeübertragungselement hat eine hohe Wärmeleitfähigkeit.

Besonders vorteilhaft ist es, wenn das Wärmeübertragungselement bei Betrieb der Heizung horizontal angeordnet ist. So kann es beispielsweise parallel zur Liegefläche eines Inkubators, in welchem die Heizung verwendet wird, angeordnet sein. Unter einer horizontalen Anordnung eines Gegenstandes wird dabei im Sinne der Erfindung verstanden, dass ein geometrischer Körper - nämlich der Gegenstand, beispielsweise die Heizung oder das Wärmeübertragungselement der Heizung - der eine dreidimensionale Gestalt hat, die eine Hauptfläche aufweist, welche größer ist als die übrigen Flächen des Körpers, räumlich derart orientiert ist, dass diese größte Fläche des Körpers parallel zu einer horizontalen Ebene ausgerichtet ist. Dabei sind selbstverständlich gewisse Toleranzwinkel vorstellbar innerhalb derer die größte Fläche abweichend von einer strikt paralleleln Ausrichtung gegenüber der horizontalen Ebene geneigt sein kann. Beispielsweise kann ein solcher Toleranzwinkel ±45° oder weniger, ±40° oder weniger, ±35° oder weniger, ±30° oder weniger, ±25° oder weniger, +20° oder weniger, ±15° oder weniger, ±10° oder weniger, ±5° oder weniger oder ±1° oder weniger betragen. Vorstellbar sind jedoch auch Toleranzwinkel von mehr als ±45°. Vorteilhaft ist es dabei, wenn die Fläche stets derart ausgerichtet ist, dass bei Betrieb der Heizung eine über der Heizung in einem Inkubator angeordnete Liegefläche für ein Kind gleichmäßig erwärmbar ist.

Die Gehäusestruktur der Heizung kann beispielsweise aus einem oberen Gehäuseelement und einem unteren Gehäuseelement bestehen. Die Gehäuseelemente liegen einander gegenüber und können eben sein oder sie können ganz oder teilweise gekrümmt sein. Jedes Gehäuseelement hat eine Oberseite und eine Unterseite. Dabei stellt die Unterseite des oberen Gehäuseelementes die obere Gehäusefläche dar und die Oberseite des unteren Gehäuseelementes stellt die untere Gehäusefläche dar. Die obere Gehäusefläche und die untere Gehäusefläche begrenzen einen zwischen den beiden Gehäuseelementen der Gehäusestruktur ausgebildeten Raum, nämlich den Strömungsraum.

Das obere Gehäuseelement ist beispielsweise ein Spritzgussteil, das bei erfindungsgemäßem Gebrauch der Heizung im Inkubator unterhalb der Liegefläche angeordnet sein kann. Es kann sich sowohl in Längs- als auch in Querrichtung von einer Seitenwand zu einer gegenüberliegenden Seitenwand des Inkubators erstrecken. Es ist jedoch auch vorstellbar, dass es kleiner ist.

Das untere Gehäuseelement kann ganz oder teilweise durch das Wärmeübertragungselement gebildet werden. Beispielsweise kann es sich dabei einfach um eine Metallplatte oder um eine Platte aus einem anderen gut- bzw. hochwärmeleitfähigen Material handeln, die mit einem gewissen Abstand parallel zur oberen Platte angeordnet ist. Vorstellbar ist auch, dass das untere Gehäuseelement einen Rahmen in Form eines Spritzgussteils (Spritzgussrahmen) aufweist, in welchem das Wärmeübertragungselement gehalten wird. Denkbar ist auch ein durchgehendes Spritzgussteil, das das untere Gehäuseelement bildet und auf welchem das Wärmeübertragungselement angeordnet ist. Sowohl der Spritzgussrahmen als auch das Wärmeübertragungselement können sich dabei - wie auch das obere Gehäuseelement - bei erfindungsgemäßem Gebrauch der Heizung sowohl in Längs- als auch in Querrichtung von einer Seitenwand zu einer gegenüberliegenden Seitenwand des Inkubators erstrecken.

Das Wärmeübertragungselement kann mit einem oder mehreren Heizelementen in wärmeleitendem Kontakt stehen. Beispielsweise können eine oder mehrere Heizpatronen unterhalb des Wärmeübertragungselementes angeordnet sein. Die von dem wenigstens einen Heizelement abgegebenen Wärme wird auf das Wärmeübertragungselement übertragen. Durch die bevorzugt sehr gute Wärmeleitfähigkeit des Materials, aus dem das Wärmeübertragungselement besteht - oder mit anderen Worten: durch die hohe Wärmeleitfähigkeit des Wärmeübertragungselementes - erwärmt sich das Wärmeübertragungselement schnell und gleichmäßig. Das so erwärmte Wärmeübertragungselement gibt wiederum die Wärme an die mit ihm in Kontakt befindliche Luft ab, beispielsweise an einen über das Wärmeübertragungselement hinwegströmenden Luftstrom. Die Wärme kann beispielsweise auch durch eine Folienheizung zugeführt werden, die auf die Unterseite des Wärmeübertragungselements aufgeklebt wird. Die Wärme kann auch durch ein auf das Wärmeübertragungselement aufgetragenes Heizelement, beispielsweise eine Dickschichtheizung, eine Dünnschichtheizung oder eine Folienheizung, übertragen werden. Eine solche Heizung kann beispielsweise durch Plasmaspritzen oder auch durch ein anderes Beschichtungsverfahren hergestellt sein.

Zwischen dem oberen und dem unteren Gehäuseelement ist das Lüfterrad angeordnet. Das Lüfterrad bewirkt eine Umwälzung der Luft im Strömungsraum der Heizung. Mithin kann es bewirken, dass ein Luftstrom entsteht, der an dem Wärmeübertragungselement entlang strömt. Der Luftstrom kann zudem durch den wenigstens einen Lüfteinlass in die Heizung, insbesondere in den Strömungsraum, hineingesaugt werden. Der Luftstrom kann auch durch den Luftauslass aus der Heizung, insbesondere aus dem Strömungsraum, herausgeblasen werden. Dabei wird im Sinne der vorliegenden Erfindung ein Luftstrom der vom Lufteinlass zum Lüfterrad strömt als anströmender Luftstrom bezeichnet, während ein Luftstrom, der vom Lüfterrad zum Luftauslass strömt, als abströmender Luftstrom bezeichnet wird. Bei erfindungsgemäßem Gebrauch der Heizung handelt es sich bei dem anströmenden Luftstrom um Luft, die aus dem Bereich des Inkubators, in dem sich das zu behandelnde Kind befindet (Inkubatorkammer, auch als Kompartment bezeichnet), zur Heizung zurückgeführt wird. Bei dem abströmenden Luftstrom handelt es sich dagegen um die Luft, die als erwärmte Luft wieder zurück in diesen Bereich geführt werden soll.

Der Lufteinlass und der Luftauslass sind im einfachsten Fall Öffnungen, beispielsweise Schlitze, die an den Seiten der Heizung zwischen dem oberen und dem unteren Gehäuseelement ausgebildet sind. Beispielsweise können solche Schlitze entstehen, indem das obere Gehäuseelement und das untere Gehäuseelement in einem gewissen Abstand zueinander angeordnet sind. Vorstellbar sind selbstverständlich auch andere Lösungen, etwa Ausnehmungen in der Gehäusestruktur oder dergleichen. Bevorzugt sind der Lufteinlass und der Luftauslass derart in der Heizung angeordnet, dass sowohl der anströmende als auch der abströmende Luftstrom über das Wärmeübertragungselement geleitet werden. Das sorgt für eine gleichmäßige Wärmeverteilung und erleichtert die Herstellung einer gleichmäßigen Temperaturverteilung in der Inkubatorkammer.

Man erkennt insofern, dass es besonders vorteilhaft ist, wenn die von dem Heizelement erzeugte Wärme mit Hilfe des Wärmeübertragungselementes sowohl auf den anströmenden Luftstrom als auch auf den abströmenden Luftstrom übertragbar ist. Das kann beispielsweise dadurch realisiert werden, dass die Fläche des Wärmeübertragungselementes so groß ist, dass sowohl der anströmende Luftstrom als auch der abströmende Luftstrom über das Wärmeübertragungselement strömen. Beispielsweise kann das Wärmeübertragungselement - wie oben bereits beschrieben - ganz oder teilweise die untere Gehäusefläche bilden. Besonders vorteilhaft ist dabei, dass das Wärmeübertragungselement eine sehr große Oberfläche aufweist. In der Folge können die zur Leistungsübertragung erforderlichen Oberflächentemperaturen deutlich unter 100°C gesenkt werden. In der Folge wird das Risiko von Verbrennungen oder unerwünschten Geruchsentwicklungen deutlich gesenkt.

In einer besonders vorteilhaften Ausführungsform ist vorgesehen, dass die Heizung ein Strömungselement aufweist. Ein solches Strömungselement kann beispielsweise dazu dienen, die Luftführung des abströmenden bzw. anströmenden Luftstromes in der Heizung möglichst optimal zu gestalten. Insbesondere kann das Strömungselement dazu dienen, Verwirbelungen und damit Vermischungen des anströmenden und abströmenden Luftstromes zu verhindern.

Das Strömungselement ist beispielsweise derart ausgebildet, dass es eine Strömungsführung für den anströmenden Luftstrom und für den abströmenden Luftstrom bildet. Bevorzugt bewirkt diese Strömungsführung, dass es zwischen dem anströmenden Luftstrom und dem abströmenden Luftstrom weder zu Verwirbelungen noch zu Kurzschlüssen kommt. Mit anderen Worten, das Strömungselement ist derart ausgebildet, dass es einen Strömungskurzschluss zwischen dem anströmenden Luftstrom und dem abströmenden Luftstrom verhindert. Besonders günstig ist es dabei, wenn das Strömungselement zwischen der oberen Gehäusefläche und der unteren Gehäusefläche angeordnet ist.

Das Strömungselement hat bevorzugt eine Oberseite und eine Unterseite. Dabei ist die Oberseite zumindest abschnittsweise der oberen Gehäusefläche zugewandt und die Unterseite ist der unteren Gehäusefläche zugewandt. Das Strömungselement kann auch einen Teil des oberen Gehäuseelementes und mithin einen Teil der oberen Gehäusefläche bilden. Beispielsweise kann das obere Gehäuseelement zweiteilig ausgebildet sein und ein Deckelelement sowie das Strömungselement umfassen. Dabei kann das Strömungselement mit dem oberen Gehäuseelement und/oder mit dem unteren Gehäuseelement einstückig ausgebildet sein.

Das Strömungselement unterteilt den Strömungsraum der Heizung in einen Anströmraum und einen Abströmraum. Der Anströmraum ist dabei der Bereich des Strömungsraumes in welchem der anströmende Luftstrom vom Lufteinlass zum Lüfterrad strömt. Der Abströmraum ist dabei der Bereich des Strömungsraumes in dem der abströmende Luftstrom vom Lüfterrad zum Luftauslass strömt. Das Lüfterrad kann dabei bevorzugt ganz oder teilweise im Abströmraum angeordnet sein.

Der Anströmraum wird dabei wenigstens von der oberen Gehäusefläche, der unteren Gehäusefläche und der Oberseite des Strömungselementes begrenzt. Der Abströmraum wird, zumindest teilweise von der Unterseite des Strömungselementes und der unteren Gehäusefläche begrenzt. Insbesondere ist es vorteilhaft, wenn das Strömungselement derart ausgebildet ist, dass es geeignet ist, den vom Lufteinlass anströmenden Luftstrom zum Lüfterrad zu leiten und gleichzeitig den vom Lüfterrad abströmenden Luftstrom zum Luftauslass zu leiten. In diesem Fall strömt die- beispielsweise aus der Inkubatorkammer kommende - anströmende Luft (der anströmende Luftstrom) vom Lufteinlass durch den Anströmraum zum Lüfterrad hin. Gleichzeitig strömt die abströmende Luft durch den Abströmraum vom Lüfterrad wieder weg zum Luftauslass und zurück in die Inkubatorkammer. Man erkennt, dass die untere Gehäusefläche sowohl den Anströmraum als auch der Abströmraum begrenzt. Mithin kann auch das Wärmeübertragungselement sowohl den Anströmraum als auch den Abströmraum begrenzen.

In der Folge strömt auch hier sowohl die anströmende Luft als auch die abströmende Luft über das Wärmeübertragungselement. Auf diese Weise kann die Luft im gesamten Strömungsraum sehr effektiv und gleichmäßig erwärmt werden. Mit anderen Worten kann sowohl die Unterseite des Anströmraumes als auch die Unterseite des Abströmraumes durch das Wärmeübertragungselement gebildet werden. In der Folge ist es ausreichend, das Wärmeübertragungselement mit einer relativ geringen Oberflächentemperatur, die deutlich unter 100°C liegen kann, zu betreiben. So sind beispielsweise Oberflächentemperaturen von 90°C oder weniger, 80°C oder weniger, 70°C oder weniger oder sogar 60°C oder weniger vorstellbar.

Es ist weiterhin von Vorteil, wenn das Strömungselement wenigstens eine Durchtrittsöffnung aufweist, durch welche der Anströmraum und der Abströmraum miteinander strömungsverbunden sind. Durch die Durchtrittsöffnung hindurch gelangt der anströmende Luftstrom zum Abströmraum, in welchem bevorzugt das Lüfterrad angeordnet ist. Beispielsweise kann die Durchtrittsöffnung direkt über dem Lüfterrad ausgebildet sein, so dass das Lüfterrad die anströmende Luft durch die Durchtrittsöffnung hindurch in den Abströmraum saugt.

Das Strömungselement bildet bevorzugt wenigstens eine Anströmbarriere für den anströmenden Luftstrom. Dabei wird der anströmende Luftstrom über die Anströmbarriere hinweg zur Durchtrittsöffnung geleitet. Die Anströmbarriere wird bevorzugt durch die Oberfläche des Strömungselementes gebildet. Dazu steht wenigstens eine Kante des Strömungselementes zumindest abschnittsweise in formschlüssigem Kontakt mit der unteren Gehäusefläche. Dabei können auf der unteren Gehäusefläche Dichtelemente vorhanden sein. Bei den Dichtelementen kann es sich beispielsweise um Vorsprünge handeln, die der Form der unteren Kante des Strömungselementes formangepasst sind. Der anströmende Luftstrom kann auf diese Weise nicht zwischen dem Strömungselement und der unteren Gehäusefläche hindurchströmen, sondern wird dazu gezwungen, über die Oberfläche des Strömungselementes hinweg zu strömen. Dabei wird der anströmende Luftstrom automatisch zur Durchtrittsöffnung geleitet.

Besonders vorteilhaft ist es dabei, wenn - wie oben beschrieben - die untere Gehäusefläche von dem Wärmeübertragungselement der Heizung gebildet wird. Der anströmende Luftstrom wird auf diese Weise bereits beim Anströmen erhitzt und strömt als vorgewärmter Luftstrom über die Oberfläche des Strömungselementes zum Lüfterrad, d.h. zum Abströmraum.

Das Strömungselement bildet weiterhin bevorzugt wenigstens eine Ausströmführung für den abströmenden Luftstrom. Die Ausströmführung wird bevorzugt durch die Unterseite des Strömungselementes sowie durch die untere Gehäusefläche, bevorzugt durch das Wärmeübertragungselement, begrenzt. Dabei ist das Strömungselement bevorzugt derart gewölbt, dass es an den bereits genannten Anströmbarrieren mit der unteren Gehäusefläche in formschlüssigem, dichtem Kontakt steht. Im Bereich der Ausströmführungen ist dagegen ein Spalt zwischen der unteren Gehäusefläche und dem Strömungselement ausgebildet. Die Ausströmführung ist bevorzugt quer zur Anströmbarriere angeordnet. Der abströmende Luftstrom kann dann durch den Spalt zwischen dem Strömungselement und der unteren Gehäusefläche wieder austreten und im Anschluss durch den Luftauslass aus der Heizung heraus und beispielsweise in die Inkubatorkammer hinein strömen. Selbstverständlich kann das Strömungselement auch mehrere Anströmbarrieren und/oder mehrere Ausströmführungen aufweisen. Unabhängig von der Anzahl der Anströmbarrieren bzw. Ausströmführungen ist das Strömungselement stets so ausgebildet, dass ein Strömungskurzschluss zwischen dem entlang der oder den Anströmbarriere/n strömenden anströmenden Luftstrom und dem entlang der oder den Ausströmführungen strömenden abströmenden Luftstrom strömenden vermeidbar ist.

In einer bevorzugten Ausführungsvariante kann das oben beschriebene Strömungselement auch noch einen Befestigungsabschnitt aufweisen. Dies ist insbesondere dann vorteilhaft, wenn das Strömungselement wie oben beschrieben einen Teil des oberen Gehäuseelementes bildet. Der Befestigungsabschnitt erstreckt sich bevorzugt parallel zur unteren Gehäusefläche bis zum Rand der Heizung. Der Befestigungsabschnitt des Strömungselementes kann beispielsweise ein Bereich des Strömungselementes sein, der sich bis zu einer oder mehreren Seitenwänden des Inkubators erstreckt. Beispielsweise kann das obere Gehäuseelement derart zweiteilig ausgebildet sein, dass es das Strömungselement aufweist und zusätzlich ein Deckelelement aufweist. Sowohl das Strömungselement als auch das Deckelelement weisen dann jeweils eine Oberseite und eine Unterseite auf. Bevorzugt ist das obere Gehäuseelement dann derart ausgebildet, dass die Unterseite des Strömungselementes und die Unterseite des Deckelementes die obere Gehäusefläche bilden, die den Strömungsraum begrenzt.

Der Anströmraum kann bei einer solchen Ausführungsform zwei Abschnitte aufweisen. Der erste Abschnitt wird von der Unterseite des Strömungselementes und der unteren Gehäusefläche, d.h. dem Wärmeübertragungselement, begrenzt. Der zweite Abschnitt wird von der Oberseite des Strömungselementes und der obere Gehäusefläche begrenzt. Im Strömungselement kann dann wenigstens eine zusätzliche Durchtrittsöffnung ausgebildet sein, durch welche der anströmende Luftstrom vom ersten Abschnitt des Anströmraumes in den zweiten Abschnitt des Anströmraumes gelangen kann. Im Bereich einer solchen zusätzlichen Durchtrittsöffnung ist jeweils eine Anströmbarriere, wie oben beschrieben, ausgebildet. Der anströmende Luftstrom strömt dann beispielsweise zuerst durch den ersten Abschnitt und wird dabei durch das Wärmeübertragungselement vorgewärmt. Er strömt dann weiter entlang der Oberfläche des Strömungselementes über die Anströmbarriere durch die zusätzliche Durchtrittsöffnung in den zweiten Abschnitt. Im Bereich des zweiten Abschnittes ist dann die Durchtrittsöffnung ausgebildet, die den Anströmraum und den Abströmraum miteinander verbindet. Durch diese Durchtrittsöffnung strömt der anströmende Luftstrom schließlich in den Abströmraum.

Man erkennt mithin, dass der Anströmraum einen oder mehrere Abschnitte aufweisen kann. Dabei wird ein Anströmraum, der nur aus einem Abschnitt besteht, von der unteren . Gehäusefläche, der Oberseite des Strömungselementes und der oberen Gehäusefläche begrenzt. Bei einem Anströmraum, der aus mehreren Abschnitten besteht, wird ein erster Abschnitt bevorzugt von der unteren Gehäusefläche und der Unterseites des Strömungselementes - nämlich im Bereich des Befestigungsabschnittes des Strömungselementes - begrenzt und ein zweiter Abschnitt wird von der Oberseite des Strömungselements und der oberen Gehäusefläche begrenzt.

Unabhängig von der konkreten Ausgestaltung der Gehäusestruktur, ist es vorteilhaft, wenn die Luftführung in der Heizung, d.h. die Führung des anströmenden und des abströmenden Luftstromes, symmetrisch ausgebildet ist. Dabei kann beispielsweise jeweils ein anströmender Luftstrom von zwei einander gegenüberliegenden Seiten in die Heizung einströmen. Gleichzeitig kann an zwei einander ebenfalls gegenüberliegenden Seiten, die jedoch quer zu den zuvor genannten Seiten angeordnet sind, jeweils ein abströmender Luftstrom die Heizung verlassen. Vorteilhaft ist es dabei, wenn das Lüfterrad zentrisch in der Heizung angeordnet ist. Wenn ein Strömungselement vorhanden ist, so ist auch dieses bevorzugt zentrisch in der Heizung angeordnet. Auch die Anströmbarrieren und die Ausströmführungen sind - soweit vorhanden - bevorzugt symmetrisch ausgebildet.

In einer weiteren bevorzugten Ausführungsform weist die erfindungsgemäße, oben beschriebene Heizung eine beheizte Kondensatsammelstelle auf. Dabei kann es sich um eine leichte Krümmung der unteren Gehäusefläche handeln. Diese Krümmung kann beispielsweise den tiefsten geometrischen Punkt des Abströmraumes darstellen. In diesem tiefsten geometrischen Punkt kann sich Kondensat sammeln, das sich bei hoher Feuchte an den Seitenwänden bilden und gegebenenfalls in das untere Gehäuse fließen kann. Üblicherweise bildet solches Kondensat ein unhygienisches Potential, weil sich in der Flüssigkeit Keime entwickeln können. Mit Hilfe der erfindungsgemäßen Konderisatsammelstelle wird dem jedoch entgegengetreten, da sich das dort sammelnde Kondensat durch das Wärmeübertragungselement wieder erhitzt, und dadurch verdunstet. Der Keimbildung wird damit wirksam vorgebeugt gegebenenfalls können Keime auch direkt abgetötet werden.

In einem weiteren Aspekt betrifft die Erfindung einen Inkubator für Kleinkinder mit einer erfindungsgemäßen, oben beschriebenen Heizung, wobei der Inkubator eine Inkubatorkammer und eine Liegefläche aufweist. Unter einem Inkubator wird dabei im Sinne der Erfindung eine geschlossene Pflegeeinheit oder eine Hybrid-Pflegeeinheit, die sowohl für die offene als auch für die geschlossene Pflege verwendbar ist, verstanden. Eine solche Pflegeeinheit weist typischerweise eine Liegefläche auf, die von Seitenwänden umrahmt ist. Der Inkubator weist weiterhin eine Abdeckhaube auf, die bei Hybrid-Pflegeeinheiten abnehmbar, verschiebbar oder verschwenkbar sein kann. Die Liegefläche und die Seitenwände bilden dabei die Inkubatorkammer, die im geschlossenen Betrieb nach oben von der Abdeckhaube begrenzt wird. Die Liegefläche bildet dabei stets den Boden der Inkubatorkammer und mithin die untere Begrenzung der Inkubatorkammer. Die erfindungsgemäße, oben beschriebene Heizung ist bei einem erfindungsgemäßen Inkubator unterhalb der Inkubatorkammer angeordnet. Bevorzugt ist dabei nicht nur das Wärmeübertragungselement horizontal angeordnet, sondern die gesamte Heizung. Unter einer horizontalen Anordnung wird auch hier verstanden, dass ein geometrischer Körper - nämlich der Gegenstand, beispielsweise die Heizung oder das Wärmeübertragungselement der Heizung - der eine dreidimensionale Gestalt hat, die eine Hauptfläche aufweist, welche größer ist als die übrigen Flächen des Körpers, räumlich derart orientiert ist, dass diese größte Fläche des Körpers parallel zu einer horizontalen Ebene ausgerichtet ist. Dabei sind selbstverständlich auch hier gewisse Toleranzwinkel - wie oben beschrieben - vorstellbar innerhalb derer die größte Fläche abweichend von einer strikt paralleleln Ausrichtung gegenüber der horizontalen Ebene geneigt sein kann.

Besonders vorteilhaft ist es dabei, wenn das obere Gehäuseelement der Heizung unterhalb der Liegefläche angeordnet ist. Die Heizung erwärmt auf diese Weise nicht nur den durch die Gehäusekonstruktion strömenden Luftstrom, sondern strahlt auch direkt Wärme auf die Liegefläche ab. Es kann insofern nicht nur die Umgebungsluft innerhalb der Inkubatorkammer vorteilhaft erwärmt und kontrolliert werden, sondern es kann auch die Liegefläche, auf welcher das Kind liegt, auf eine vorteilhafte Temperatur gebracht werden.

In diesem Sinne ist es ebenfalls vorteilhaft, wenn die Heizung parallel zur Liegefläche angeordnet ist. Auf diese Weise kann die Liegefläche sehr gleichmäßig erwärmt werden. Dabei kann die Liegefläche zu Therapiezwecken auch in gewissen Grenzen schräggestellt werden - beispielsweise für eine Trendelenburg- oder Anti-Trendelenburg-Lagerung. Die Position der Heizung verändert sich dann jedoch nicht, sondern sie bleibt parallel zur horizontalen Ausgangsposition der Liegefläche. Auch ist es vorstellbar, dass der Abstand der Liegefläche zur Heizung variabel ist. Beispielsweise kann die Liegefläche zur Pflege des darauf befindlichen Kindes angehoben oder abgesenkt werden, ohne dass die Position der Heizung verändert wird.

In einer weiteren vorteilhaften Ausführungsform ist vorgesehen, dass der oben beschriebene Inkubator eine Grundfläche mit einer Länge L und einer Breite B hat, wobei die Länge I und Breite b der Fläche des Wärmeübertragungselementes der Heizung im Wesentlichen der Größe der Länge und Breite der Grundfläche des Inkubators entspricht. Unter der Grundfläche des Inkubators wird dabei wiederum die Projektionsfläche des Inkubators bei einer senkrechten Aufsicht von oben verstanden. Man erkennt, dass sich das Wärmeübertragungselement der Heizung im Wesentlichen über die gesamte Grundfläche des Inkubators erstreckt. Auf diese Weise kann das Wärmeübertragungselement selbst bei relativ geringen Oberflächentemperaturen eine ausreichende und gleichmäßige Erwärmung der Liegefläche und der Luft im Inkubator bewirken.

Weiterhin kann vorgesehen sein, dass für eine Luftzuführung des anströmenden Luftstromes aus der Inkubatorkammer zur Heizung an zwei einander gegenüberliegenden ersten Seiten der Liegefläche Ansaugschlitze ausgebildet sind, und das für eine Luftzuführung des abströmenden Luftstromes von der Heizung in die Inkubatorkammer Luftschlitze an zwei einander gegenüberliegenden Seiten der Liegefläche ausgebildet sind. Dabei können beispielsweise die einander gegenüberliegenden ersten Seiten der Liegefläche die Stirnseiten des Inkubators sein und die zwei einander gegenüberliegenden zweiten Seiten, die senkrecht zu diesen Stirnseiten angeordneten Längsseiten des Inkubators.

Eine besonders vorteilhafte Luftführung innerhalb des Inkubators kann sich dann wie folgt darstellen: Die im Inkubator vorhandene Luft strömt durch die Ansaugschlitze aus der Inkubatorkammer in den Bereich der Heizung. Dort strömt die Luft als anströmender Luftstrom durch den Lufteinlass in den Strömungsraum, insbesondere in den Anströmraum. Im Anströmraum wird der anströmende Luftstrom über die untere Gehäusefläche, d.h. also über die Oberfläche des Wärmeübertragungselementes bis zu der bzw. den Anströmbarrieren geleitet. Dabei wird der anströmende Luftstrom erwärmt. Von der bzw. den Anströmbarriere/n aus wird der anströmende Luftstrom weiter über die Oberfläche des Strömungselementes durch die Durchtrittsöffnung zum Lüfterrad geleitet. Dabei kann der anströmende Luftstrom wenn er entlang der Anströmbarrieren strömt durch die zusätzlichen Durchtrittsöffnungen strömen, sofern diese vorhanden sind. Bei Durchtritt durch die Durchtrittsöffnung, die den Anströmraum und den Abströmraum miteinander verbindet, gelangt der anströmende Luftstrom zum Lüfterrad. Dabei wird der anströmende Luftstrom zu abströmenden Luftstrom. Von dem Lüfterrad aus wird die nun abströmende Luft als abströmender Luftstrom ebenfalls über die untere Gehäusefläche entlang der Unterseite des Strömungselementes zur Ausstromführung geleitet und von dort zum Luftauslass. Dabei wird auch der abströmende Luftstrom erwärmt. Vom Luftauslass gelangt die abströmende Luft durch die Luftschlitze wieder zurück in die Inkubatorkammer. In der Inkubatorkammer kann die so erwärmte Luft beispielsweise an den Seitenwänden der Inkubatorkammer vorhangartig nach oben steigen. Während des Nachobensteigens kann sich die Luft wieder leicht abkühlen. An der Decke der Inkubatorkammer - also beispielsweise entlang der Abdeckhaube - strömt die sich abkühlende Luft dann wieder entlang bis zu den Seitenflächen, an welchen Sie wieder in die Heizung gesaugt werden soll und wieder absinkt. Dieser absinkende Luftstrom würde nun wiederum durch die Ansaugschlitze zur Heizung zurückgeführt und der Kreislauf beginnt erneut.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Figur 1: eine schematische Darstellung einer erfindungsgemäßen Heizung für einen Inkubator für Kleinkinder,
- Figur 2: eine schematische Aufsicht auf die untere Gehäusefläche der erfindungsgemäßen Heizung von Figur 1 mit schematischer Darstellung des anströmenden und des abströmenden Luftstromes,
- Figur 3: eine schematische Aufsicht auf eine untere Gehäusefläche einer weiteren Ausführungsform einer erfindungsgemäßen Heizung mit Strömungselement,
- Figur 4a: einen schematischen Querschnitt durch einen erfindungsgemäßen Inkubator mit einer Heizung gemäß Figur 3 entlang der Schnittlinie A/A von Figur 3,
- Figur 4b: einen schematischen Querschnitt durch einen erfindungsgemäßen Inkubator mit einer Heizung gemäß Figur 3 entlang der Querschnittslinie C/C,
- Figur 5a: den schematischen Strömungsweg eines anströmenden Luftstromes in einem Inkubator entsprechend Figur 4a,
- Figur 5b: den schematischen Strömungsweg eines abströmenden Luftstromes in einem erfindungsgemäßen Inkubator entsprechend 4b,
- Figur 6: eine Explosionszeichnung einer Ausführungsform einer erfindungsgemäßen Heizung.

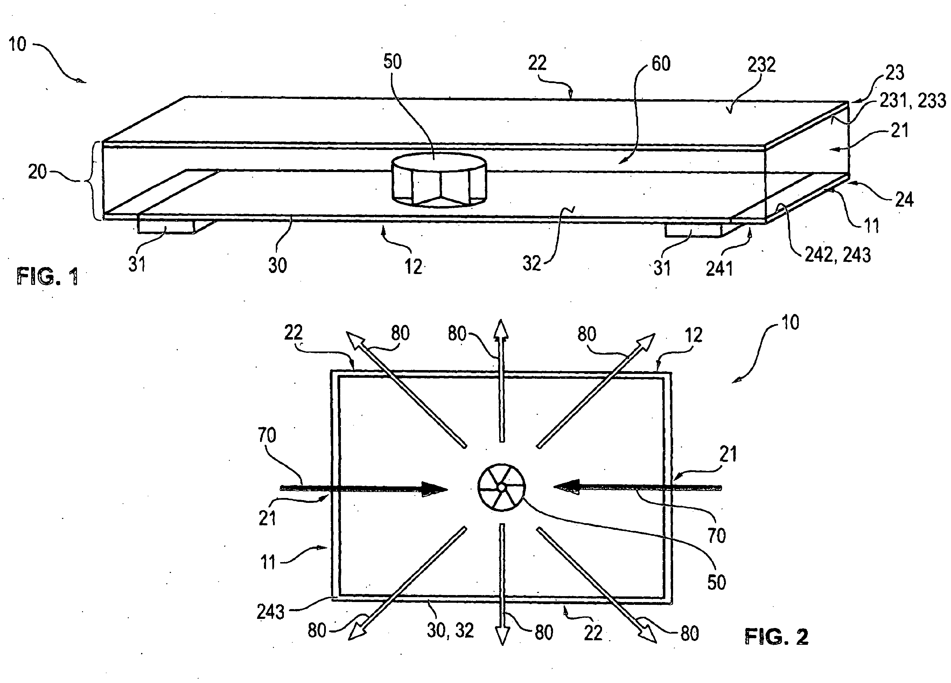

Man erkennt in Figur 1, dass eine erfindungsgemäße Heizung 10 eine Gehäusestruktur 20 mit einem oberen Gehäuseelement 23 und einem unteren Gehäuseelement 24 umfasst. Das obere Gehäuseelement 23 hat eine Unterseite 231 und eine Oberseite 232. Die Unterseite 231 des oberen Gehäuseelementes 23 bildet eine obere Gehäusefläche 233. Das untere Gehäuseelement 24 hat eine Unterseite 241 und eine Oberseite 242. Die Oberseite 242 des unteren Gehäuselementes 24 bildet eine untere Gehäusefläche 243. Die untere Gehäusefläche 243 kann sich dabei wie im dargestellten, schematischen Beispiel entlang der gesamten Oberseite 242 des unteren Gehäuseelementes 24 erstrecken. Es ist aber auch vorstellbar, dass sich die untere Gehäusefläche 243 nur entlang eines Abschnittes des unteren Gehäuseelementes 24 erstreckt. Die obere Gehäusefläche 233 kann sich dabei wie im dargestellten, schematischen Beispiel entlang der gesamten Unterseite 231 des oberen Gehäuseelementes 23 erstrecken. Es ist aber auch vorstellbar, dass sich die obere Gehäusefläche 233 nur entlang eines Abschnittes des oberen Gehäuseelementes 24 erstreckt.

Die obere Gehäusefläche 233 und die untere Gehäusefläche 243 begrenzen einen Strömungsraum 60. Bei Betrieb der Heizung begrenzt die obere Gehäusefläche 233 den Strömungsraum 60 von oben und die untere Gehäusefläche 243 begrenzt den Strömungsraum 60 von unten. Die obere Gehäusefläche 233, mithin also die Unterseite 231 des oberen Gehäuseelementes 23, könnte mithin auch als "obere Fläche 233, die den Strömungsraum 60 begrenzt," bezeichnet werden. Die untere Gehäusefläche 243, mithin also die Oberseite 232 des unteren Gehäuseelementes 24, könnte mithin auch als "untere Fläche 243, die den Strömungsraum 60 begrenzt," bezeichnet werden.

An der unteren Gehäusefläche 243 ist das Wärmeübertragungselement 30 angeordnet. Unter dem Wärmeübertragungselement 30 sind im dargestellten Beispiel zwei Heizelemente 31 angeordnet. Diese stehen in wärmeleitendem Kontakt mit dem Wärmeübertragungselement 30. Vorstellbar ist jedoch auch, dass eine erfindungsgemäße Heizung 10 nur ein Heizelement 31 aufweisen kann. Auch können mehr als zwei Heizelemente 31 vorhanden sein. Das oder die Heizelemente 31 können auch flächenförmig ausgebildet sein. Das Wärmeübertragungselement 30 ist flächenförmig und hat eine ebene Oberfläche 32. Bei Betrieb der Heizung 10 ist das Wärmeübertragungselement 30 horizontal angeordnet.

Im Strömungsraum 60 ist weiterhin ein Lüfterrad 50 angeordnet. Dieses ist im dargestellten Beispiel mittig auf dem Wärmeübertragungselement 30 angeordnet. Es ist jedoch auch vorstellbar, dass das Lüfterrad 50 dezentral an einer der Seiten der Gehäusestruktur 20 angeordnet ist.

Die erfindungsgemäße Heizung 10 weist weiterhin einen Lufteinlass 21 und einen Luftauslass 22 auf. Der Lufteinlass 21 ist an einer ersten Seite 11 der Gehäusestruktur 20 ausgebildet. Der Luftauslass 22 ist an einer zweiten Seite 12 der Gehäusestruktur 20 ausgebildet. Die Seite 11, an welcher der Lufteinlass 21 ausgebildet ist, und die Seite 12, an welcher der Luftauslass 22 ausgebildet ist, sind quer zueinander ausgerichtet.

Man erkennt weiterhin in Figur 1, dass das Wärmeübertragungselement 30 ganz oder teilweise die untere Gehäusefläche 243 bildet. Insbesondere bildet die Oberfläche 32 des Wärmeübertragungselementes 30 einen Teil der unteren Gehäusefläche 243. Dabei kann sich das Wärmeübertragungselement 30 - wie im dargestellten Beispiel ersichtlich - bis kurz vor die Kante der Seite 11 erstrecken oder sogar bis ganz an die Kante der Seite 12 oder umgekehrt. Selbstverständlich kann sich das Wärmeübertragungselement 30 in einer nicht dargestellten Variante auch an beiden Seiten bis an den Rand des unteren Gehäuseelementes 24 erstrecken oder es kann an beiden Seiten ein Abstand zum Rand des Gehäuseelementes 24 ausgebildet sein.

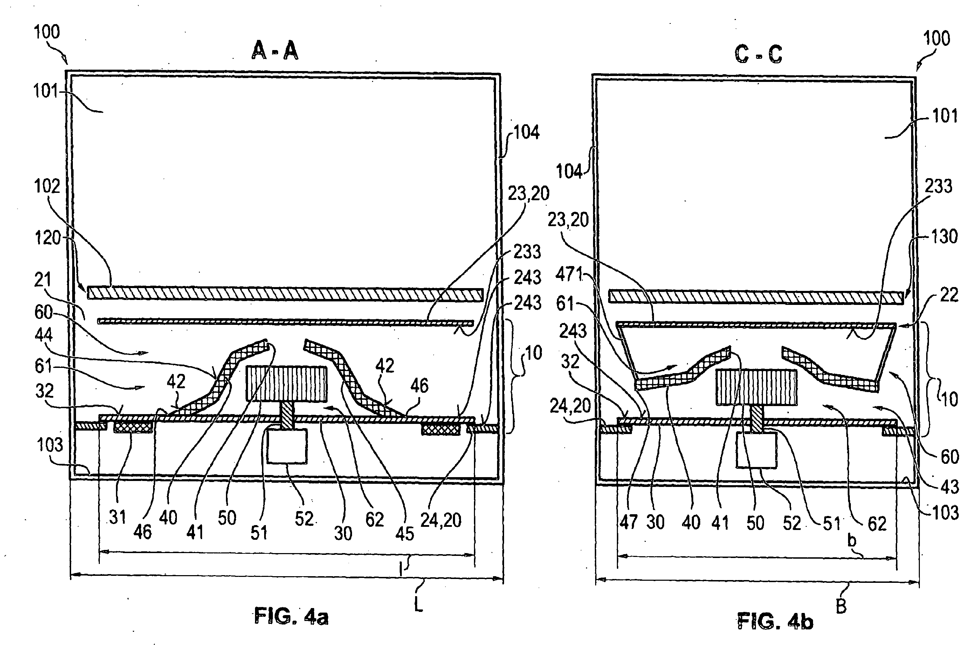

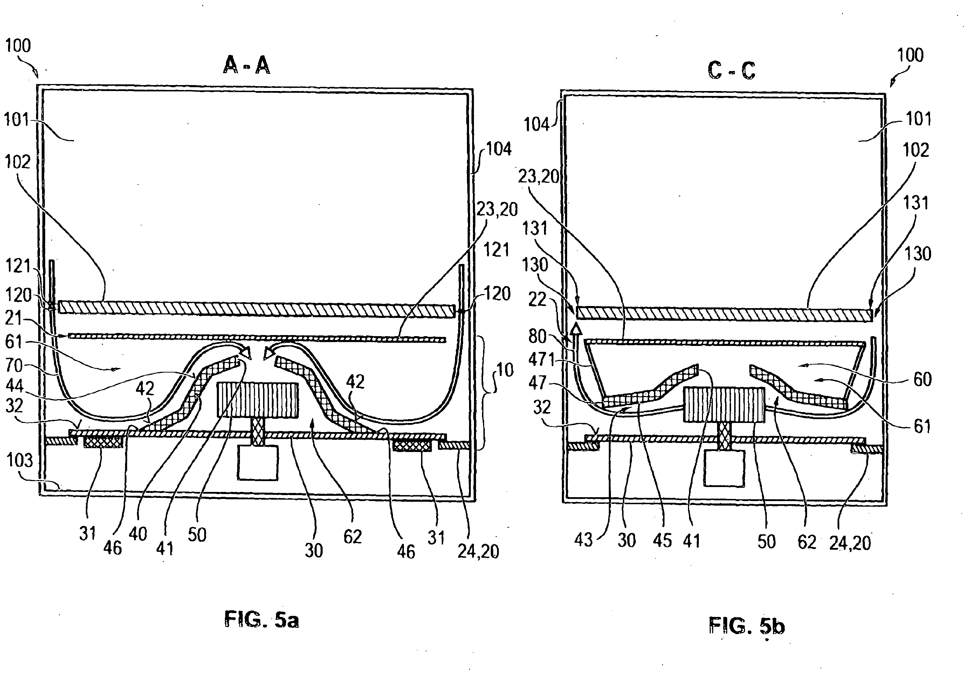

In Figur 2 erkannt man, dass sowohl der einströmende Luftstrom 70 als auch der abströmende Luftstrom 80 über die Oberfläche 32 des Wärmeübertragungselements 30 - mithin also über die untere Gehäusefläche 243 - geleitet wird. Insbesondere erkennt man in Figur 2, dass das Wärmeübertragungselement 30 die untere Gehäusefläche 243 der erfindungsgemäßen Heizung 10 nahezu vollständig ausfüllt. Auch hier ist in der Mitte des Wärmeübertragungselementes 30 ein Lüfterrad 50 angeordnet. Der Lufteinlass 21 befindet sich an der schmalen Seite 11 der erfindungsmäßen Heizung 10. Diese schmale Seite 11 kann bei Betrieb der Heizung beispielsweise zur Stirnseite des erfindungsgemäßen Inkubators 100 (vgl. Fig. 4a, 4b, 5a, 5b) hin orientiert sein. Der Luftauslass 22 ist an der langen Seite 12 der erfindungsgemäßen Heizung 10 ausgebildet. Diese Seite 12 kann beispielsweise der Längsseite des erfindungsgemäßen Inkubators entsprechend. Man erkennt, dass der anströmende Luftstrom 70 und der abströmende Luftstrom 80 symmetrisch ausgebildet sind. Eine weitere detaillierte Darstellung von Luftströmen in einem erfindungsgemäßen Inkubator 100 bzw. einer erfindungsgemäßen Heizung 10 ist in den Figuren 5a und 5b dargestellt.

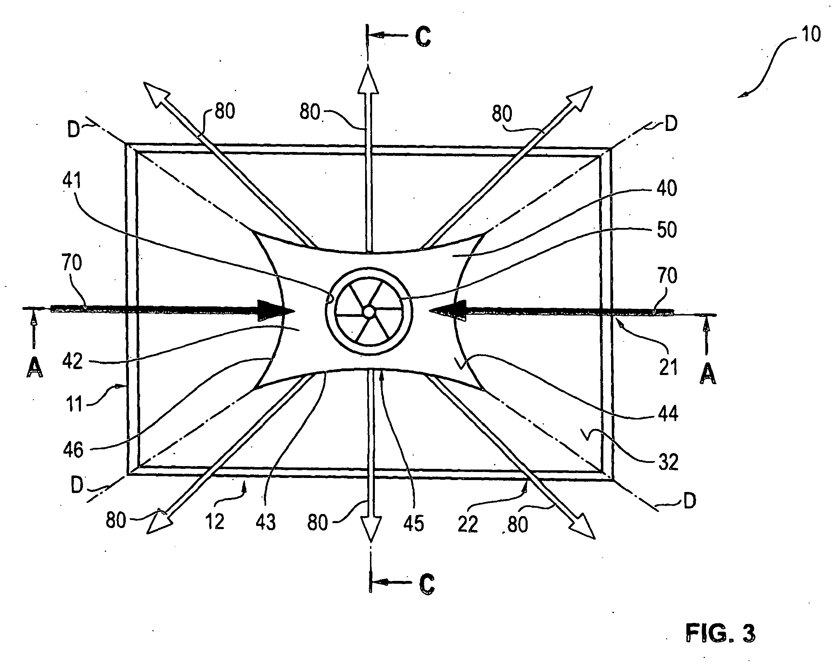

Man erkennt in Figur 3 eine weitere schematische Funktionsskizze für ein Ausführungsbeispiel der erfindungsgemäßen Heizung 10. Auch in Figur 3 blickt man von oben auf die bereits oben im Zusammenhang mit den Figuren 1 und 2 beschriebene untere Gehäusefläche 243 der ebenfalls oben bereits beschriebenen Gehäusestruktur 20 der erfindungsgemäßen Heizung 10. Das Wärmeübertragungselement 30 nimmt auch hier im Wesentlichen die gesamte Fläche der unteren Gehäusefläche 243 ein, so dass die Oberfläche 32 einen Teil der unteren Gehäusefläche 243 bildet. Zentrisch ist ein Lüfterrad 50 angeordnet. Man erkennt in Figur 3 weiterhin, dass die dort gezeigte erfindungsgemäße Heizung 10 ein Strömungselement 40 aufweist. Dieses Strömungselement 40 ist mittig über dem Wärmeübertragungselement 30 angeordnet. Es kann jedoch auch dezentral angeordnet sein.

Das Strömungselement 40 hat eine Durchtrittsöffnung 41, eine erste Kante 46 und eine zweite Kante 47. Das gezeigte Strömungselement 40 ist dabei symmetrisch ausgebildet, so dass es jeweils zwei erste Kanten 46 und zwei zweite Kanten 47 aufweist. Vorstellbar ist auch, dass das Strömungselement 40 eine asymmetrische Form aufweist wobei das Strömungselement 40 jeweils nur eine, zwei oder mehrere erste Kanten 46 und/oder zweite Kanten 47 aufweisen kann. Die Form des Strömungselementes 40 ist in den vorliegenden Figuren (vgl. Fig. 3, 4a, 4b, 5a, 5b, 6) insofern jeweils nur schematisch gezeigt, um die Funktionsweise des Strömungselementes 40 zu erläutern.

In jedem Fall sind die Form, die Anzahl und die Ausdehnung der Kanten 46 und 47 derart an die Heizung 10 angepasst, dass es zwischen dem anströmenden Luftstrom 70 und dem abströmenden Luftstrom 80 keinen Kurzschluss gibt. Das Strömungselement 40 ist mithin derart ausgebildet, dass es eine Strömungsführung für den anströmenden Luftstrom 70 und für den abströmenden Luftstrom 80 bildet. Bevorzugt bewirkt diese Strömungsführung, dass es zwischen dem anströmenden Luftstrom 70 und dem abströmenden Luftstrom 80 weder zu Verwirbelungen noch zu Kurzschlüssen kommt. Mit anderen Worten, das Strömungselement 40 ist derart ausgebildet, dass es einen Strömungskurzschluss zwischen dem anströmenden Luftstrom 70 und dem abströmenden Luftstrom 80 verhindert. Die Kanten 46, 47 des Strömungselementes 40 können sich dabei beispielsweise von der Mitte der Heizung 10 entlang einer oder entlang beider Diagonalen D des Wärmeübertragungselementes 30 erstrecken. Dabei können sie sich entlang der gesamten Diagonalen D oder nur entlang eines Teils der Diagonalen D erstrecken. Bevorzugt erstreckt sich wenigstens die Kante 46 entlang der gesamten Diagonalen D.

Die erste Kante 46 steht in dichtendem Kontakt mit dem Wärmeübertragungselement 30. Auf diese Weise bildet die Oberfläche 44 des Strömungselementes eine Anströmbarriere 42 für den anströmenden Luftstrom 70. Dieser anströmende Luftstrom 70 wird mithin, vom Lufteinlass 21 kommend, über die Oberfläche 32 des Wärmeübertragungselementes 30 und über die Oberfläche 44 des Strömungselementes 40 hin zur Durchtrittsöffnung 41 geleitet.

Die Kante 47 hat keinen Kontakt zur Oberfläche 32 des Wärmeübertragungselementes 30. Zwischen dem Strömungselement 40 und dem Wärmeübertragungselement 30 wird auf diese Weise eine Öffnung gebildet. Diese Öffnung stellt eine Ausströmführung 43 für den abströmenden Luftstrom 80 dar. Der abströmende Luftstrom 80 kommt von der Durchtrittsöffnung 41 zu dem Lüfterrad 50 und wird von dort über die Oberfäche 32 des Wärmeübertragungselementes 30 und durch die Ausströmführung 43 hindurch zum Luftauslass 22 geleitet. Der Luftauslass 22 ist an der Seite 12 angeordnet. Man erkennt, dass sowohl der anströmende Luftstrom 70 als auch der abströmende Luftstrom 80 über die Oberfläche 32 des Wärmeübertragungselementes 30 geleitet werden. Dabei stellt das Strömungselement 40 sicher, dass es nicht zu unerwünschten Verwirbelungen des anströmenden Luftstroms 70 und des abströmenden Luftstroms 80 kommt. Auf diese Weise arbeitet die erfindungsgemäße Heizung 10 besonders geräuscharm. Gleichzeitig kann die Oberfläche 32 des Wärmeübertragungselementes 30 eine relativ geringe Oberflächentemperatur, beispielsweise weniger als 100°C, aufweisen. Der anströmende Luftstrom 70 wird bereits vorgewärmt, bevor er durch die Durchtrittsöffnung 41 zum Lüfterrad 50 gelangt. In der Folge muss der abströmende Luftstrom 80 nur noch wenig Wärmeleistung aufnehmen, um die gewünschte Temperatur zu erreichen. Er kann daher mit Hilfe einer relativ geringen Oberflächentemperatur der Oberfläche 32 auf die gewünschte Lufttemperatur erhitzt werden, bevor er durch den Luftauslass 22 die Heizung 10 wieder verlässt.

Man erkennt in Figur 4a - die einen Querschnitt durch einen erfindungsgemäßen Inkubator 100 entlang der Linie A/A von Figur 3 darstellt - einen schematischen Schnitt durch eine erfindungsgemäße Heizung 10. Die Heizung 10 ist in einem erfindungsgemäßen Inkubator 100 unterhalb der Liegefläche 102, auf welcher während des Betriebes des Inkubators 100 das zu behandelnde Kind liegt, angeordnet. Man erkennt, dass die Gehäusestruktur 20 parallel zur Liegefläche 102 des Inkubators 100 angeordnet ist. Insbesondere die obere Gehäusefläche 233 und die untere Gehäusefläche 243 mit dem Wärmeübertragungselement 30 sind parallel zur Liegefläche 102 angeordnet. Darüber hinaus sind die obere Gehäusefläche 233 und die untere Gehäusefläche 243 auch parallel zum Boden 103 des inkubators angeordnet. Dabei kann die Liegefläche 102 um eine gewissen Winkel gegenüber der Heizung 10 verkippt werden kann, beispielsweise zur Ausführung eines Trendelenburg-Manövers. Dies ist insbesondere dann sinnvoll, wenn das Kind, das auf der Liegefläche liegt, gedreht werden soll. Auch ist vorstellbar, dass der Boden 103 keine plane Ebene darstellt, sondern eine Wölbung aufweist.

Man erkennt weiterhin in Figur 4a, dass die Gehäusestruktur 20 nämlich die obere Gehäusefläche 233 und die untere Gehäusefläche 243 einen Strömungsraum 60 begrenzen. Dieser Strömungsraum 60 weist einen Anströmraum 61 und einen Abströmraum 62 auf. Dabei wird der Strömungsraum 60 mit Hilfe des Strömungselementes 40 in den Anströmraum 61 und den Abströmraum 62 unterteilt. Im Strömungsraum 60, nämlich im Abströmraum 62, ist das Lüfterrad 50 der Heizung 10 angeordnet. Das Lüfterrad 50 ist über eine Achse 51 mit einem Motor 52 verbunden. Der Motor 52 bewirkt über die Achse 51 eine Drehung des Lüfterrades 50, wenn die Heizung 10 in Betrieb ist. Der Motor 52 ist dabei unterhalb der unteren Gehäusefläche 243 angeordnet.

Man erkennt weiterhin, dass die untere Gehäusefläche 243 das Wärmeübertragungselement 30 trägt. Dabei bildet das Wärmeübertragungselement 30 einen großen Teil der unteren Gehäusefläche 243. Die Oberfläche 32 des Wärmeübertragungselementes 30 bildet auch hier einen Teil der unteren Gehäusefläche 243. Unterhalb des Wärmeübertragungselementes 30 sind zwei Heizelemente 31 angeordnet. Vorstellbar ist jedoch auch, dass nur ein Heizelement 31 oder mehrere Heizelemente 31 unterhalb des Wärmeübertragungselementes 30 angeordnet sind. Bei dem Heizelement 31 handelt es sich vorzugsweise um eine Heizpatrone. Die Heizpatrone gibt ihre Wärme direkt an das Wärmeübertragungselement 30 ab. Das Wärmeübertragungselement 30 ist dabei bevorzugt derart gestaltet, dass es ein guter Wärmeleiter ist. Beispielsweise kann das Wärmeübertragungselement 30 aus Aluminium, temperaturbeständigem und gut wärmeleitendem Kunststoff oder einem sonstigen gut wärmeleitenden Material bestehen. In der Folge ist die gesamte Oberfläche 32 des Wärmeübertragungselementes 30 im Betrieb der Heizung durch die Heizelemente 31 auf eine gewisse Oberflächentemperatur gebracht.

Das in Figur 4a erkennbare Strömungselement 40 weist wie oben bereits beschrieben (vgl. Fig. 3) eine Durchtrittsöffnung 41 auf, durch welche der anströmender Luftstrom aus dem Anströmraum 61 in den Abströmraum 62 gelangen kann. Man erkennt auch hier, dass das Strömungselement 40 eine Oberfläche 44 und eine Unterseite 45 hat. Die Oberfläche 44 begrenzt den Anströmraum 61. Die Unterseite 45 begrenzt den Abströmraum 62. In der Folge wird der Anströmraum 61 mindestens durch die obere Gehäusefläche 233 die Oberfläche 32 des Wärmeübertragungselementes 30 und die Oberfläche 44 des Strömungselementes 40 begrenzt. Der Abströmraum 62 wird mindestens durch die Oberseite 32 des Wärmeübertragungselementes 30 und die Unterseite 45 des Strömungselementes 40 begrenzt.

Das Strömungselement 40 weist weiterhin - wie bereits für Fig. 3 beschrieben - eine Kante 46 auf, die in dichtem, insbesondere in dichtendem, Kontakt mit der Oberfläche 32 des Wärmeübertragungselementes 30 steht. Ein dichter Kontakt ist dabei ein Kontakt, bei dem die Kante 46 so nahe an der Oberfläche 32 des Wärmeübertragungselementes 30 ausgebildet ist, dass der anströmende Luftstrom 70 nicht zwischen der Kante 46 und der Oberfläche 32 hindurchströmt. Mit anderen Worten: Das Strömungselement 40 verhindert, dass die anströmende Luft zwischen der Oberfläche 32 des Wärmeübertragungselementes 30 und der Kante 46 des Strömungselementes 40 hindurchströmen kann. Das Strömungselement 40 bildet auf diese Weise eine Anströmbarriere 42 für den anströmenden Luftstrom.

Man erkennt weiterhin in Figur 4a, dass die Länge I des Wärmeübertragungselementes 30 im Wesentlichen der Länge L des Bodens 103 bzw. der wie oben definierten Grundfläche des Inkubators 100 entspricht.

Für den Eintritt des anströmenden Luftstromes in die Heizung 10 ist zwischen der Gehäusestruktur 20 und der Außenwand des Inkubators 100 an beiden Seiten jeweils ein Lufteinlass 21 ausgebildet. Um die Luft aus der Inkubatorkammer 101 zu diesem Lufteinlass 21 zu führen, sind an den Seite 121 der Liegefläche 102 Ansaugschlitze 120 ausgebildet.

Man erkennt in Figur 4b, die einen Querschnitt durch einen erfindungsgemäßen Inkubator 100 entlang der Linie C/C von Figur 3 darstellt, ebenfalls die zuvor beschriebene und in Fig. 4a schematisch dargestellte Heizung 10. Wie bereits dargestellt begrenzt die obere Gehäusefläche 233 und die untere Gehäusefläche 243 mit dem Wärmeübertragungselement 30 den Strömungsraum 60. In diesem Querschnitt sind an den Seiten der oberen Gehäusefläche 233 die Luftauslässe 22 dargestellt. Diese dienen der Rückführung der erwärmten Luft von der Heizung 10 zur Inkubatorkammer 101. Um die Luft von dem Luftauslass 22 zur Inkubatorkammer 101 zu führen, ist zwischen der Seite 131 der Liegefläche 102 jeweils ein Diffusionsschlitz 130 ausgebildet.

Man erkennt in Figur 4b weiterhin, dass die Kante 47 des Strömungselementes 40 keinen Kontakt zur Oberfläche 32 des Wärmeübertragungselementes 30 aufweist. Zwischen der Kante 47 und der Oberfläche 32 ist die Ausströmführung 43 ausgebildet. Durch diese Ausströmöffnung 43 kann die aus dem Abströmraum 62 kommende erwärmte Luft, nämlich der abströmende Luftstrom 80 (vgl. Fig. 5b), zu den Luftauslässen 22 strömen. Das Strömungselement 40 hat dabei eine weitere Kante 471, die im Kontakt mit der oberen Gehäusefläche 233 steht und einen strömungstechnischen Kurzschluss zwischen dem Anströmraum 61 und dem Abströmraum 62 im Bereich der Ausströmführung verhindert. Es ist jedoch auch vorstellbar, dass das Strömungselement 40 ohne diese zusätzliche Kante 471 ausgebildet ist.

Man erkennt weiterhin, dass die Breite b des Wärmeübertragungselementes 30 im Wesentlichen der Breite B der Grundfläche 103 des Inkubators 100 entspricht.

In Figur 5a erkennt man, dass der anströmende Luftstrom 70 aus der Inkubatorkammer 101 des Inkubators 100 kommt. Dabei handelt es sich um Luft, die der Heizung 10 zur erneuten Erwärmung zugeführt werden soll. Dieser anströmende Luftstrom 70 strömt an der Seite 121 der Liegefläche 102 durch einen Ansaugschlitz 120, der zwischen der Liegefläche 102 und der Wand des Inkubators 100 ausgebildet ist. Von dort strömt der anströmende Luftstrom 70 durch den Lufteinlass 21 in den Strömungsraum 60 der Heizung 10. Der Lufteinlass 21 ist zwischen dem oberen Gehäuseelement 23 und der Seitenwand 104 des Inkubators 100 ausgebildet. In einer alternativen Ausführungsform kann der Lufteinlass 21 auch zwischen dem Strömungselement 40 und dem oberen Gehäuseelement 23 ausgebildet sein. Vorstellbar ist auch, dass der Lufteinlass 21 zwischen dem Strömungselement 40 und der Gehäusewand, etwa der Seitenwand 104, des Inkubators 100 ausgebildet ist. In diesem Fall erstreckt sich wenigstens ein Teil oder Abschnitt des Strömungselementes 40 bis zur. Seitenwand 104 des Inkubators 100. In jedem Fall wird jedoch der Anströmraum 61 durch das Strömungselement 40, das obere Gehäuseelement 23, nämlich die obere Gehäusefläche 232, und die Oberfläche 32 des Wärmeübertragungselementes 30 begrenzt. Der anströmende Luftstrom 70, der in den Anströmraum 61 eingeströmt ist, wird über die Oberfläche 32 des Wärmeübertragungselementes 30 geleitet und dabei erwärmt. Der anströmende Luftstrom 70 wird dann weiter über die von dem Strömungselement 40 gebildete Anströmbarriere 42 bis hin zur Durchtrittsöffnung 41 geleitet. An der Durchtrittsöffnung 41 strömt der anströmende Luftstrom 70 zum Lüfterrad 50 hin in den Abströmraum 62.

Im Abströmraum 62 wird der zuvor anströmende Luftstrom 70 nun als abströmender Luftstrom 80 bezeichnet. Mit anderen Worten: Der anströmende Luftstrom 70 strömt durch den Anströmraum 61, der abströmende Luftstrom 80 strömt durch den Abströmraum 62 und im Bereich der Durchtrittsöffnung 41, die den Anströmraum 61 und den Abströmraum 62 mit einander verbindet, geht der anströmende Luftstrom 70 in den abströmenden Luftstrom 80 über.

In Figur. 5b erkennt man, dass dann der abströmende Luftstrom 80 vom Lüfterrad 50 über die Oberfläche 32 des Wärmeübertragungselementes 30 hinweg und unter der Unterseite 45 des Strömungselementes 40 entlang durch die Austrittsführung 43 wieder zurück zum Luftauslass 22 geleitet wird. Von dort gelangt der abströmende, erwärmte Luftstrom 80 zu Luftschlitzen 130. Diese Luftschlitze 130 sind zwischen der Seite 131 der Liegefläche 102 und der Seitenwand 105 des Inkubators 100 ausgebildet. Der ankommende erwärmte Luftstrom 80 steigt dann wie ein Vorhang an der Seitenwand 105 entlang nach oben und gelangt so in die Inkubatorkammer 101. Auch hier verhindert die optional vorhandene Kante 471 einen strömungstechnischen Kurzschluss zwischen dem Anströmraum 61 und dem Abströmraum 62.

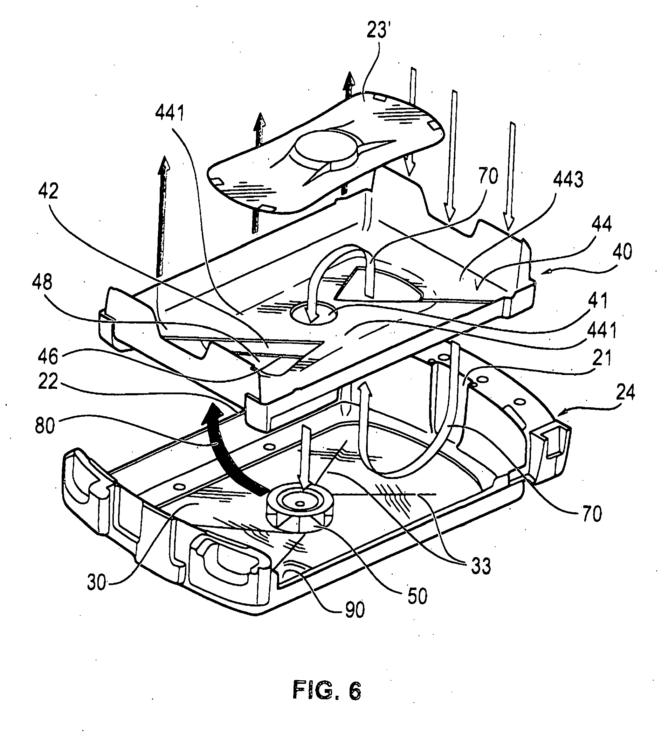

Bei dem in Figur 6 dargestellten Ausführungsbeispiel erkennt man, dass die Gehäuseelemente 23', 24, das Strömungselement 40' und das Wärmeübertragungselement 30 der Heizung 10 jeweils eine zumindest teilweise gewölbte Form aufweisen. In diesem Ausführungsbeispiel weist das Strömungselement 40' einen Befestigungsabschnitt 443 auf. Der Befestigungsabschnitt 443 umgibt einen inneren Abschnitt 441 und erstreckt sich im montierten Zustand bis zur (nicht dargestellten) Wand des Inkubators. Das Strömungselement 40' ist auch hier zwischen dem oberen Gehäuseelement 23' und dem unteren Gehäuseelement 24 angeordnet. Das obere Gehäuseelement 23' ist als Deckelelement ausgebildet und wird auf das Strömungselement 40' aufgesetzt. Mithin kann das obere Gehäuseelement 23' auch als Deckelelement 23' bezeichnet werden.

Das Strömungselement 40' weist neben der Durchtrittsöffnung 41 weiterhin zwei symmetrisch zueinander angeordnete zusätzliche Durchtrittsöffnungen 48 auf. Im Bereich dieser Durchtrittsöffnungen 48 ist jeweils eine Anströmbarriere 42 ausgebildet. Die Kanten 46 der Anströmbarrieren 42 stehen sind im montierten Zustand in formschlüssiger, dichtender Verbindung mit Dichtelementen 33, die auf dem Wärmeübertragungselement 30 ausgebildet sind.

Der Anströmraum 61 ist in diesem Ausführungsbeispiel in zwei Abschnitte unterteilt. Der erste Abschnitt wird von der Unterseite 45 des Strömungselementes 40' und der unteren Gehäusefläche 243 begrenzt. Er erstreckt sich jeweils vom Lufteinlass 21 bis zur Anströmbarriere 42. Der zweite Abschnitt wird von der Oberseite 44 des Strömungselementes 40' und der Unterseite 232 des oberen Gehäuseelementes 23' begrenzt. Er erstreckt sich von den zusätzlichen Durchtrittsöffnungen 48 bis zur Durchtrittsöffnung 41.

In dem in Figur 6 gezeigten Ausführungsbeispiel weist das Wärmeübertragungselement 30 weiterhin eine Kondensatsammelstelle 90 auf. Diese Kondensatsammelstelle 90 liegt bei Bertrieb des Inkubators 100 an einer tiefen Stelle des Inkubators 100, so dass sich bildende Flüssigkeit im Inkubator 100 zu dieser Stelle hinunter fließen kann. So erkennt man in Fig. 6, dass es sich bei der Kondensatsammelstelle 90 um eine leichte Vertiefung in der Oberfläche 32 des Wärmeübertragungselementes 30 handelt. Die Vertiefung ist knickartig ausgebildet und stellt insofern kein Strömungshindemis für den anströmenden oder für den abströmenden Luftstrom 70, 80 dar. In dieser Vertiefung, d.h. in der Kondensatsammelstelle 90 kann möglicherweise anfallendes Kondensat zusammenfließen, da es sich um den tiefsten Punkt der Oberfläche 32 des Wärmeübertragungselementes 30 handelt. Da diese Oberfläche 32 jedoch erfindungsgemäß beheizt ist, kann das Kondensat dort rasch wieder verdunsten. Auch ist die Oberfläche 32 des Wärmeübertragungselementes 30 einfach abwischbar, beispielsweise wenn die Heizung 10 zu Reinigungszwecken auseinander gebaut wird, so dass Kondensat, das sich möglicherweise dort gesammelt hat, einfach abgewischt werden kann.

In jedem Fall erkennt man, dass es bei einer Heizung 10 für einen Inkubator für Kleinkinder 100, wobei die Heizung 10 eine Gehäusestruktur 20 mit einer oberen Gehäusefläche 233 und einer unteren Gehäusefläche 243, wenigstens einen Lufteinlass 21 für einen anströmenden Luftstrom 70, wenigstens einen Luftauslass 22 für einen abströmenden Luftstrom 80, wenigstens ein Lüfterrad 50, wenigstens ein Heizelement 31 und wenigstens ein Wärmeübertragungselement 30 ausweist, wobei die obere Gehäusefläche 233 und die untere Gehäusefläche 243 einen Strömungsraum 60 begrenzen, vorteilhaft ist, wenn das Wärmeübertragungselement 30 eine bei Betrieb der Heizung horizontal angeordnete Fläche mit einer ebenen Oberfläche 32 ist. Besonders günstig ist es dabei auch, wenn von dem Heizelement 31 erzeugte Wärme mit Hilfe des Wärmeübertragungselementes 30 sowohl auf den anströmenden Luftstrom 70 als auch auf den abströmenden Luftstrom 80 übertragbar ist. Dabei kann das Wärmeübertragungselement 30 ganz oder teilweise die untere Gehäusefläche 24 bilden.

Man erkennt weiterhin, dass es vorteilhaft ist, wenn die Heizung 10 ein Strömungselement 40, 40' aufweist. Dabei ist es besonders günstig, wenn das Strömungselement 40 den Strömungsraum 60 in einen Anströmraum 61 und einen Abströmraum 62 unterteilt. Erfindungsgemäß kann dabei das Strömungselement 40 zwischen der oberen Gehäusefläche 233 und der unteren Gehäusefläche 243 angeordnet sein. Beispielsweise kann das Strömungselement 40, 40' mit einem oberen Gehäuseelement 23 und/oder mit einem unteren Gehäuseelement 24 einstückig ausgebildet sein.

Besonders zweckmäßig ist es, wenn das Strömungselement 40, 40' derart ausgebildet ist, dass es geeignet ist, den von Lufteinlass 21 anströmenden Luftstrom 70 zum Lüfterrad zu leiten und gleichzeitig den vom Lüfterrad 50 abströmenden Luftstrom 80 zum Luftauslass 22 zu leiten. Zweckmäßig ist es, wenn das Strömungselement 40, 40' eine Durchtrittsöffnung 41 aufweist, durch welche der Anströmraum 61 und der Abströmraum 62 mit einander strömungsverbunden sind.

Weiterhin erkennt man, dass es von Vorteil ist, wenn die Heizung 10 eine geheizte Kondensatsammelstelle 90 aufweist.

Man erkennt weiterhin, dass es vorteilhaft ist, wenn ein Inkubator für Kleinkinder 100 mit einer oben beschriebenen Heizung 10 eine Inkubatorkammer 101 und eine Liegefläche 102 aufweist. Dabei ist es zweckmäßig, wenn die obere Gehäusefläche 23 der Heizung 10 unterhalb der Liegefläche 102 angeordnet ist. Weiterhin ist es zweckmäßig, wenn die Heizung 10 parallel zur Liegefläche 102 angeordnet ist. Besonders vorteilhaft ist es, wenn der Inkubator 100 eine Grundfläche 103 mit einer Länge L und einer Breite B hat, wobei die Länge I und die Breite b der Fläche des Wärmeübertragungselementes 30 der Heizung 10 im Wesentlichen der Größe der Länge L und Breite B der Grundfläche 103 des Inkubators 100 entsprechen.

Man erkennt weiter, dass es zweckmäßig ist, wenn für eine Luftzuführung des anströmenden Luftstromes 70 aus der Inkubatorkammer 101 zur Heizung 10 an zwei einander gegenüberliegenden ersten Seiten 121 der Liegefläche 102 Ansaugschlitze 120 ausgebildet sind und wenn für eine Luftzuführung des abströmenden Luftstromes 80 von der Heizung 10 in die Inkubatorkammer 101 Luftschlitze 130 an zwei einander gegenüberliegenden zweiten Seiten 131 der Liegefläche 102 ausgebildet sind.

**BEZUGSZEICHENLISTE**

| | | | |
|---|---|---|---|
| L | Länge | B | Breite |
| l | Länge | b | Breite |
| | | | |
| 10 | Heizung | | |
| 11 | Seite | | |
| 12 | Seite | | |
| | | | |
| 20 | Gehäusestruktur | | |
| 21 | Lufteinlass | | |
| 22 | Luftauslass | | |
| 23, 23' | oberes Gehäuseelement | | |
| 231 | Unterseite | | |
| 232 | Oberseite | | |
| 233 | obere Gehäusefläche | | |
| 24 | unteres Gehäuseelement | | |
| 241 | Unterseite | | |
| 242 | Oberseite | | |
| 243 | untere Gehäusefläche | | |
| | | | |
| 30 | Wärmeübertragungselement | | |
| 31 | Heizelement | | |
| 32 | Oberfläche | | |
| 33 | Dichtelement | | |
| | | | |
| 40, 40' | Strömungselement | | |
| 41 | Durchtrittsöffnung | | |
| 42 | Anströmbarriere | | |
| 43 | Ausströmführung | | |
| 44 | Oberfläche | | |
| 441 | Abschnitt | | |
| 443 | Befestigungsabschnitt | | |
| 45 | Unterseite | | |
| 46 | Kante | | |
| 47 | Kante | | |
| 471 | Kante | | |
| 48 | Durchtrittsöffnung | | |
| 50 | Lüfterrad | | |
| 51 | Achse | | |
| 52 | Motor | | |
| | | | |
| 60 | Strömungsraum | | |
| 61 | Anströmraum | | |
| 62 | Abströmraum | | |
| | | | |
| 70 | anströmender Luftstrom | | |
| 80 | abströmender Luftstrom | | |
| 100 | Inkubator | | |
| 101 | Inkubatorkammer | | |
| 102 | Liegefläche | | |
| 103 | Boden | | |
| 104 | Seitenwand | | |
| 105 | Seitenwand | | |
| | | | |
| 120 | Ansaugschlitze | | |
| 121 | Seite | | |
| | | | |
| 130 | Luftschlitz | | |
| 131 | Seite | | |

## Patentansprüche

1. Inkubator für Kleinkinder (100) wobei der Inkubator (100) eine Inkubatorkammer (101), eine Liegefläche (102) und eine Heizung (10) aufweist, wobei die Heizung (10) eine Gehäusestruktur (20) mit einer oberen Gehäusefläche (233) und einer unteren Gehäusefläche (243), wenigstens einen Lufteinlass (21) für einen anströmenden Luftstrom (70), wenigstens einen Luftauslass (22) für einen abströmenden Luftstrom (80), wenigstens ein Lüfterrad (50), wenigstens ein Heizelement (31) und wenigstens ein Wärmeübertragungselement (30) aufweist, wobei die obere Gehäusefläche (233) und die untere Gehäusefläche (243) einen Strömungsraum (60) begrenzen, **dadurch gekennzeichnet, dass** das Wärmeübertragungselement (30) eine bei Betrieb der Heizung horizontal angeordnete Platte mit einer ebenen Oberfläche (32) ist, und dass von dem Heizelement (31) erzeugte Wärme mit Hilfe des Wärmeübertragungselementes (30) sowohl auf den anströmenden Luftstrom (70), der vom Lufteinlass (21) zum Lüfterrad (50) strömt, als auch auf den abströmenden Luftstrom (80), der vom Lüfterrad (50) zum Luftauslass (22) strömt, übertragbar ist, wobei sowohl der anströmende Luftstrom (70) als auch der abströmende Luftstrom (80) über die Oberfläche (32) des Wärmeübertragungselementes (30) geleitet wird.

2. Inkubator gemäß Anspruch 1, **dadurch gekennzeichnet, dass** das Wärmeübertragungselement (30) ganz oder teilweise die untere Gehäusefläche (243) bildet.

3. Inkubator gemäß wenigstens einem der vorhergehenden Ansprüche, **dadurch gekennzeichnet, dass** die Heizung (10) ein Strömungselement (40, 40') aufweist.

4. Inkubator gemäß Anspruch 3, **dadurch gekennzeichnet, dass** das Strömungselement (40, 40') zwischen der oberen Gehäusefläche (233) und der unteren Gehäusefläche (243) angeordnet ist.

5. Inkubator gemäß einem der Ansprüche 3 oder 4, **dadurch gekennzeichnet, dass** das Strömungselement (40, 40') mit einem oberen Gehäuseelement (23, 23') und/oder mit einem unteren Gehäuseelement (24) einstückig ausgebildet ist.

6. Inkubator gemäß wenigstens einem der Ansprüche 3 bis 5, **dadurch gekennzeichnet, dass** das Strömungselement (40, 40') den Strömungsraum (60) in einen Anströmraum (61) und einen Abströmraum (62) unterteilt.

7. Inkubator gemäß wenigstens einem der Ansprüche 3 bis 6, **dadurch gekennzeichnet, dass** das Strömungselement (40, 40') derart ausgebildet ist, dass es geeignet ist, den vom Lufteinlass (21) anströmenden Luftstrom (70) zum Lüfterrad (50) zu leiten und gleichzeitig den vom Lüfterrad (50) abströmenden Luftstrom (80) zum Luftauslass (22) zu leiten.

8. Inkubator gemäß wenigstens einem der Ansprüche 3 bis 7, **dadurch gekennzeichnet, dass** das Lüfterrad (50) ganz oder teilweise im Abströmraum angeordnet ist.

9. Inkubator gemäß wenigstens einem der Ansprüche 3 bis 8, **dadurch gekennzeichnet, dass** das Strömungselement (40) wenigstens eine Anströmbarriere (42) für den anströmenden Luftstrom bildet.

10. Inkubator gemäß wenigstens einem der Ansprüche 3 bis 9, **dadurch gekennzeichnet, dass** das Strömungselement (40) wenigstens eine Ausströmführung (43) für den abströmenden Luftstrom bildet.

11. Inkubator gemäß Anspruch 10, **dadurch gekennzeichnet, dass** die Ausströmführung (43) quer zur Anströmbarriere (42) angeordnet ist.

12. Inkubator gemäß wenigstens einem der Ansprüche 3 bis 11, **dadurch gekennzeichnet, dass** das Strömungselement (40, 40') eine Durchtrittsöffnung (41) aufweist, durch welche der Anströmraum (61) und der Abströmraum (62) miteinander strömungsverbunden sind.

13. Inkubator gemäß wenigstens einem der vorhergehenden Ansprüche, **dadurch gekennzeichnet, dass** die Heizung (10) eine beheizte Kondensatsammelstelle (90) aufweist.

14. Inkubator gemäß einem der Ansprüche 1 bis 13, **dadurch gekennzeichnet, dass** der Inkubator (100) eine Grundfläche mit einer Länger (L) und einer Breite (B) hat, wobei die Länge (l) und Breite (b) der Fläche des Wärmeübertragungselementes (30) der Heizung (10) im Wesentlichen Größe der Länge (L) und Breite (B) der Grundfläche (103) des Inkubators (100) entspricht.

15. Inkubator gemäß wenigstens einem der Ansprüche 1 bis 14, **dadurch gekennzeichnet, dass** für eine Luftzuführung des anströmenden Luftstromes (70) aus der Inkubatorkammer (101) zur Heizung (10) an zwei einander gegenüberliegenden ersten Seiten (121) der Liegefläche (102) Ansaugschlitze (120) ausgebildet sind und dass für eine Luftzuführung des abströmenden Luftstromes (80) von der Heizung (10) in die Inkubatorkammer (101) Diffusorschlitze (130) an zwei einander gegenüberliegenden zweiten Seiten (131) der Liegefläche (103) ausgebildet sind.

## Claims

1. An incubator for infants (100), wherein the incubator (100) has an incubator chamber (101), a reclining surface (102) and a heater (10), wherein the heater (10) has a housing structure (20) with an upper housing surface (233) and a lower housing surface (243), at least one air inlet (21) for an inflowing air stream (70), at least one air outlet (22) for an outflowing air stream (80), at least one fan impeller (50), at least one heating element (31), and at least one heat-transfer element (30), wherein the upper housing surface (233) and the lower housing surface (243) define a flow space (60), **characterised in that** the heat-transfer element (30) is a plate arranged horizontally during the operation of the heater with a flat surface (32), and **in that** heat generated by the heating element (31) can be transferred with the aid of the heat-transfer element (30) to both the inflowing air stream (70), flowing from the air inlet (21) to the fan impeller (50), and the outflowing air stream (80), flowing from the fan impeller (50) to the air outlet (22), wherein both the inflowing air stream (70) and the outflowing air stream (80) are guided by way of the surface (32) of the heat-transfer element (30).

2. An incubator according to claim 1, **characterised in that** the heat-transfer element (30) forms the lower housing surface (243) entirely or partially.

3. An incubator according to at least one of the preceding claims, **characterised in that** the heater (10) has a flow element (40, 40').

4. An incubator according to claim 3, **characterised in that** the flow element (40, 40') is arranged between the upper housing surface (233) and the lower housing surface (243).

5. An incubator according to one of claims 3 or 4, **characterised in that** the flow element (40, 40') is formed in one piece with an upper housing element (23, 23') and/or with a lower housing element (24).

6. An incubator according to at least one of claims 3 to 5, **characterised in that** the flow element (40, 40') divides the flow space (60) into an inflow space (61) and an outflow space (62).

7. An incubator according to at least one of claims 3 to 6, **characterised in that** the flow element (40, 40') is formed so that it is suitable for guiding the air stream (70) flowing in from the air inlet (21) to the fan impeller (50) and for guiding at the same time the air stream (80) flowing out from the fan impeller (50) to the air outlet (22).

8. An incubator according to at least one of claims 3 to 7, **characterised in that** the fan impeller (50) is arranged entirely or partially in the outflow space.

9. An incubator according to at least one of claims 3 to 8, **characterised in that** the flow element (40) forms at least one inflow barrier (42) for the inflowing air stream.

10. An incubator according to at least one of claims 3 to 9, **characterised in that** the flow element (40) forms at least one outflow guide (43) for the outflowing air stream.

11. An incubator according to claim 10, **characterised in that** the outflow guide (43) is arranged transversely to the inflow barrier (42).

12. An incubator according to at least one of claims 3 to 11, **characterised in that** the flow element (40, 40') has a passage opening (41), through which the inflow space (61) and the outflow space (62) are connected with one another for flow.

13. An incubator according to one of the preceding claims, **characterised in that** the heater (10) has a heated condensate-collection site (90).

14. An incubator according to one of claims 1 to 13, **characterised in that** the incubator (100) has a base with a length (L) and a width (B), wherein the length (1) and width (b) of the surface of the heat-transfer element (30) of the heater (10) substantially correspond to the size of the length (L) and width (B) of the base (103) of the incubator (100).

15. An incubator according to at least one of claims 1 to 14, **characterised in that** suction slots (120) are formed on two mutually opposite first sides (121) of the reclining surface (102) for feeding air to the inflowing air stream (70) from the incubator chamber (101) to the heater (10), and **in that** diffuser slots (130) are formed on two mutually opposite second sides (131) of the reclining surface (103) for feeding air to the outflowing air stream (80) from the heater (10) into the incubator chamber (101).

## Revendications

1. Incubateur pour enfants en bas âge (100), l'incubateur (100) présentant une chambre d'incubateur (101), une surface de couchage (102) et un dispositif de chauffage (10), le dispositif de chauffage (10) présentant une structure de boîtier (20) avec une surface de boîtier supérieure (233) et une surface de boîtier inférieure (243), au moins une entrée d'air (21) pour un flux d'air entrant (70), au moins une sortie d'air (22) pour un flux d'air sortant (80), au moins une roue de ventilateur (50), au moins un élément chauffant (31) et au moins un élément de transfert thermique (30), la surface de boîtier supérieure (233) et la surface de boîtier inférieure (243) délimitant un espace d'écoulement (60), **caractérisé en ce que** l'élément de transfert thermique (30) est une plaque disposée horizontalement pendant le fonctionnement du dispositif de chauffage, avec une surface plane (32) et **en ce que** la chaleur générée par l'élément chauffant (31) peut être transférée à l'aide de l'élément de transfert thermique (30) à la fois au flux d'air entrant (70) qui s'écoule depuis l'entrée d'air (21) jusqu'à la roue de ventilateur (50) et au flux d'air sortant (80) qui s'écoule depuis la roue de ventilateur (50) vers la sortie d'air (22), le flux d'air entrant (70) ainsi que le flux d'air sortant (80) étant guidés par le biais de la surface (32) de l'élément de transfert thermique (30).

2. Incubateur selon la revendication 1, **caractérisé en ce que** l'élément de transfert thermique (30) forme complètement ou en partie la surface de boîtier inférieure (243).

3. Incubateur selon au moins l'une quelconque des revendications précédentes, **caractérisé en ce que** le dispositif de chauffage (10) présente un élément d'écoulement (40, 40').

4. Incubateur selon la revendication 3, **caractérisé en ce que** l'élément d'écoulement (40, 40') est disposé entre la surface de boîtier supérieure (233) et la surface de boîtier inférieure (243).

5. Incubateur selon l'une quelconque des revendications 3 ou 4, **caractérisé en ce que** l'élément d'écoulement (40, 40') est réalisé d'une seule pièce avec un élément de boîtier supérieur (23, 23') et/ou avec un élément de boîtier inférieur (24) .

6. Incubateur selon au moins l'une quelconque des revendications 3 à 5, **caractérisé en ce que** l'élément d'écoulement (40, 40') divise l'espace d'écoulement (60) en un espace d'entrée d'écoulement (61) et un espace de sortie d'écoulement (62).

7. Incubateur selon au moins l'une quelconque des revendications 3 à 6, **caractérisé en ce que** l'élément d'écoulement (40, 40') est réalisé de telle sorte qu'il soit apte à conduire le flux d'air (70) arrivant depuis l'entrée d'air (21) vers la roue de ventilateur (50) et de conduire simultanément le flux d'air (80) sortant de la roue de ventilateur (50) vers la sortie d'air (22).

8. Incubateur selon au moins l'une quelconque des revendications 3 à 7, **caractérisé en ce que** la roue de ventilateur (50) est disposée complètement ou en partie dans l'espace de sortie d'écoulement.

9. Incubateur selon au moins l'une quelconque des revendications 3 à 8, **caractérisé en ce que** l'élément d'écoulement (40) forme au moins une barrière à l'entrée de l'écoulement (42) pour le flux d'air entrant.

10. Incubateur selon au moins l'une quelconque des revendications 3 à 9, **caractérisé en ce que** l'élément d'écoulement (40) forme au moins une barrière à la sortie de l'écoulement (43) pour le flux d'air sortant.

11. Incubateur selon la revendication 10, **caractérisé en ce que** le guidage de sortie d'écoulement (43) est disposé transversalement à la barrière à l'entrée de l'écoulement (42).

12. Incubateur selon au moins l'une quelconque des revendications 3 à 11, **caractérisé en ce que** l'élément d'écoulement (40, 40') présente une ouverture de passage (41) à travers laquelle l'espace d'entrée d'écoulement (61) et l'espace de sortie d'écoulement (62) sont connectés fluidiquement l'un à l'autre.

13. Incubateur selon au moins l'une quelconque des revendications précédentes, **caractérisé en ce que** le dispositif de chauffage (10) présente un point de collecte de condensat (90).

14. Incubateur selon l'une quelconque des revendications 1 à 13, **caractérisé en ce que** l'incubateur (100) présente une surface de base avec une longueur (L) et une largeur (B), la longueur (l) et la largeur (b) de la surface de l'élément de transfert thermique (30) du dispositif de chauffage (10) correspondant essentiellement à la longueur (L) et la largeur (B) de la surface de base (103) de l'incubateur (100).

15. Incubateur selon au moins l'une quelconque des revendications 1 à 14, **caractérisé en ce que** des fentes d'aspiration (120) sont réalisées pour une alimentation en air du flux d'air entrant (70) depuis la chambre d'incubateur (101) dans le dispositif de chauffage (10) au niveau de deux premiers côtés opposés l'un à l'autre (121) de la surface de couchage (102) et **en ce que** des fentes de diffuseur (130) sont réalisées au niveau de deux deuxièmes côtés opposés l'un à l'autre (131) de la surface de couchage (103) pour une alimentation en air du flux d'air sortant (80) depuis le dispositif de chauffage (10) dans la chambre d'incubateur (101) .
